Europäisches Patentamt

European Patent Office

Office européen des brevets

(19)

(11) **EP 0 869 800 B1**

(12) **FASCICULE DE BREVET EUROPEEN**

(45) Date de publication et mention
de la délivrance du brevet:
**29.08.2001 Bulletin 2001/35**

(51) Int Cl.⁷: **A61K 33/30**

(21) Numéro de dépôt: **96943178.2**

(86) Numéro de dépôt international:
**PCT/FR96/02062**

(22) Date de dépôt: **23.12.1996**

(87) Numéro de publication internationale:
**WO 97/24131 (10.07.1997 Gazette 1997/30)**

(54) **COMPOSITION ANTIBACTERIENNE A BASE D'ACYLGLYCINE ET DE SEL DE ZINC**

ANTIBAKTERIELLES MITTEL AUF ACYLGLYCIN- UND ZINKSALZ-BASIS

ACYLGLYCINE- AND ZINC SALT-BASED ANTIBACTERIAL COMPOUND

(84) Etats contractants désignés:
**BE CH DE ES FR GB IT LI LU MC**

(30) Priorité: **28.12.1995 FR 9515651**

(43) Date de publication de la demande:
**14.10.1998 Bulletin 1998/42**

(73) Titulaire: **PIERRE FABRE DERMO-COSMETIQUE
92100 Boulogne (FR)**

(72) Inventeurs:
• **MSIKA, Philippe**
  **F-75018 Paris (FR)**
• **COUTELLE, Hervé**
  **F-31860 Labarthe-sur-Lèze (FR)**
• **LAGARDE, Isabelle**
  **F-31540 Maurens (FR)**

(74) Mandataire: **Warcoin, Jacques**
  **Cabinet Régimbeau**
  **20, rue de Chazelles**
  **75847 Paris cedex 17 (FR)**

(56) Documents cités:
**DE-A- 3 443 985          FR-A- 2 192 795
FR-A- 2 224 169**

**Description**

La présente invention concerne une composition antibactérienne utilisable aussi bien en tant que médicament ou produit cosmétique, qu'en tant qu'agent désinfectant de tout type de matériel, notamment du matériel chirurgical.

Dans l'état de la technique antérieure, les acylaminoacides, en particulier les dérivés de $C_1$ à $C_{30}$ acylés avec un ou plusieurs acides aminés ont déjà été proposés dans diverses applications antimicrobiennes, antifongiques et antiparasitaires. La demande EP 601 911 concerne en plus des propriétés citées, des soins en hygiène et agriculture. La demande FR 2 698 869 concerne le procédé de préparation à partir d'une protéine sécrétée par le *Bombix mori*. La demande WO9221318 revendique l'utilisation à partir d'hydrolysats de protéines de céréales dans le but de préparer les détergents de diverses surfaces (sols, textiles).

FR 222 41 69 décrit des compositions à propriétés bactéricides, fongicides et antivirales comportant, à titre de principe actif, au moins un lipoaminoacide résultant de la combinaison d'un acide gras en $C_6$-$C_{12}$ avec un aminoacide. Ce document n'enseigne cependant pas la combinaison avec un autre principe actif tel que le zinc.

De manière inattendue, la Demanderesse a maintenant trouvé que les acylglycines en $C_6$ à $C_{10}$ sont potentialisés en présence d'un sel de zinc.

Cette activité est particulièrement marquée sur les bactéries à gram positifs tels les staphylocoques, les streptocoques et les corynébactéries.

Les gram positifs ont un portage cutané universel, avec un essaimage très rapide du fait de leur habitat ubiquitaire. Ils ont de plus un taux de multiplication exponentiel dès qu'ils se situent dans un milieu favorable. Par exemple, le Staphylocoque doré constitue un problème car ce germe est responsable d'infections nocosomiales exposant les sujets infectés à des staphylococcies récidivantes. Les streptocoques sont des germes impliqués dans les caries dentaires, dans les angines (streptocoques) et sont des témoins de contaminations fécales lors de leur mise en évidence lors de contrôles microbiologiques des eaux.

Les corynébactéries appartiennent à la flore physiologique cutanée mais certaines espèces peuvent devenir des contaminants gênants producteurs d'odeurs tels les *C.xerosis* ou *C.minutissimum* au niveau des aisselles ou des pieds.

Les propriétés antimicrobiennes de molécules peuvent se quantifier par l'étude de l'inhibition de la multiplication des germes (bactériostase) ou par l'étude de la bactéricide.

Dans le cas de la bactériostase, généralement de faibles quantités sont nécessaires pour obtenir une efficacité inhibitrice : de l'ordre de la dizaine de microgrammes.

La bactéricidie, par contre, nécessite généralement de fortes concentrations. Ceci est compensé par le fait que le temps de contact entre le germe et le produit est très court, suivi généralement d'un lavage.

De nombreuses molécules sont utilisées et formulées dans un but curatif : lotions antiseptiques de divers dérivés cationiques ou iodés de confort plus ou moins agréable pour le patient en particulier pour le petit enfant.

De plus, certaines petites plaies cutanées ont besoin d'une action mécanique et moussante afin de faire partir de petits débris tels que cellules cutanées mortes, poussières et petits cailloux, suite à un petit traumatisme par exemple.

Il existe donc un réel besoin pour un produit topique à propriétés antigram+ à cinétique d'activité rapide qui posséderait en plus des propriétés trophiques pour les peaux lésées telles celles vues lors de dermatites atopiques ou de petites lésions acnéiques surinfectées.

C'est pourquoi la présente invention a pour objet l'utilisation d'une acylglycine en $C_6$ à $C_{10}$ couplée à un sel de zinc pour la préparation d'un médicament ou cosmétique topique ayant une activité bactériostatique et bactéricide.

Le médicament topique ou cosmétique antibactérien selon l'invention se présentera de préférence sous forme d'une composition pharmaceutique ou cosmétique comportant les excipients adaptés pour une application topique ou sur des muqueuses (buccale, vaginale, rectale).

Les compositions antibactériennes selon l'invention trouvent également leur application dans le domaine de la désinfection de différents types de matériel.

De préférence, on choisira d'une part l'octanoylglycine et d'autre part le sulfate de zinc.

Dans les compositions conformes à l'invention, la concentration en acylglycine, en particulier en octanoylglycine, est de préférence située dans l'intervalle compris entre 0,1 et 10%, de préférence entre 2 et 4%. Le sulfate de zinc sera présent à des concentrations comprises entre 0,05 et 5%, de préférence entre 0,5 et 1%. Ces concentrations sont exprimées en pourcentage en poids par rapport au poids total de la composition.

Le niveau de l'efficacité a été prouvé pour des souches gram+ en particulier pour le staphylocoque doré (*Staphylococcus aureus* 6538 P). L'efficacité bactéricide de la formule a pu être chiffrée. En effet, les formules avec les deux actifs et avec un seul des deux actifs se distinguent de l'excipient non bactéricide.

Les exemples qui suivent sont destinés à illustrer l'invention sans aucunement en limiter la portée.

MATERIEL ET METHODE:

. <u>souche</u>: *Staphylococcus aureus* 6538 P

- <u>milieu de culture</u> :

  . bouillon et gélose trypticase soja (Biomérieux)
  . bouillon Trypticase soja + 10% Tween 80.

- <u>appareillage</u> :

  . microplaques stériles Nunc R Nunclon à 96 puits
  . eau distillée stérile
  . tubes à essais stériles
  . étuve à 32°C (Jouan)
  . spectrophotomètre PRIM et cuves adaptées
  . billes de verres stériles

- <u>produits</u> :
  Les solutions-mères des produits cités ci-dessous ont été préparés comme suit :

  . Zn SO 4: solution aqueuse à 5%
  . n-octanoyl-glycine : solution à 1% dans 30% butylène glycol puis chauffage jusqu'à dissolution homogène.

- <u>méthodes</u> :

  - <u>CMI</u> : Concentration Minimale Inhibitrice
    Une distribution de 100 µl de bouillon Trypticase soja est réalisée dans chaque cupule de la microplaque stérile A. Une distribution de 100 µl des différents produits a lieu dans une cupule (on est alors à 50% d'actif) (une cupule par produit). Une série de dilutions sériées au demi est ensuite réalisée sur microplaque.
    Un témoin stérilité du milieu et un autre témoin de la vigueur de la souche sont réalisés en parallèle sur la microplaque.
    L'ensemencement de la microplaque (A) a lieu sous la hotte après préparation de l'inoculum bactérien comme suit :
    Les souches ayant été préalablement repiquées la veille sur gélose Trypticase soja, un inoculum $10^8$ germes/ml est dispersé dans l'eau distillée stérile puis agité au vortex. Les billes de verre assurant une bonne dispersion des souches, la mesure de la densité optique permet de préparer un inoculum adéquat par rapport à un étalonnage pré-établi et validé.
    La répartition de la souche a lieu dans une deuxième microplaque (B) stérile par distribution de 100 µl d'inoculum par microcupule.
    L'ensemencement a lieu par un repiquage de la microplaque (B) vers la microplaque (A), il se réalise ainsi une dilution au 1/100 de l'inoculum : la microplaque A contenant alors $10^6$ germes par ml, peut être mise à l'étuve 24 h à 32°C.
    La lecture de la plus grande dilution donnant lieu à une clarté identique au témoin stérilité du milieu, constitue la concentration minimale inhibitrice ou CMI.

<u>Etude de l'association</u>

- n-octanoyl-glycine - Zn SO 4

Des solutions-mères à 2 fois la CMI sont réalisées (comme précédemment décrit).
La méthode de l'échiquier réalisée sur microplaque est utilisée pour croiser les séries de dilutions au demi de chaque actif.

Après ensemencement et incubation à 32°C, la lecture des CMI est réalisée.

Un repérage des CMI donnant lieu à une synergie, et une additivité peut alors se faire.

Nous obtenons après calcul des FiC Index le tableau suivant

| Produits | CMI | | FiC | FiC Index |
| | Seuls | Associés | | |
|---|---|---|---|---|
| Zn SO 4 | 0,15 | 0,03 | 0,20 | |
| | | | | 0,70 |
| n octanoyl glycine | 0,25 | 0,125 | 0,5 | |

Le calcul des FiC Index des produits est la somme FiC des rapports (FiC A + FiC B).

Le FiC d'un produit A est défini comme suit :

$$\text{FiCA} = \frac{\text{CMI du produit A en association}}{\text{CMI du produit A seul}}$$

L'association de deux produits A et B est synergique si le FiC Index est inférieur ou égal à 0,75. Plus la valeur FiC est faible, plus la synergie est importante.

On considère qu'il y a simple additivité pour des valeurs de FiC comprises entre 0,75 et 1,1 et indifférence dans l'intervalle compris entre 1,1 et 2. Au-delà, l'association est antagoniste.

Les efficacités bactéricides de formules avec et sans actif ont été déterminées sur *St.aureus* ATCC 9144 pour un temps de contact de 2 et 5 minutes (selon le protocole décrit à la Pharmacopée Française).

Les produits ont été testés purs (à 90%) à 50% et à 23%.

Les résultats exprimés en chute logarithmique du nombre de germes montrent que le produit pur dès cinq minutes de contact, a une activité antiseptique (réduction logarithmique supérieure à 5).

D'une manière générale, le produit met 1'24 pour réduire la population microbienne de 1 Log 10 (= D Value = 1'24).

Le tableau suivant nous permet de résumer les cinétiques d'efficacité d'une formulation à base de n-octanoylglycine et de sulfate de zinc.

D. Value sur *St.aureus* 6538 P

| | D. Value | r |
|---|---|---|
| Excipient | 1 h 54' | 0,88 |
| formule | 1'24 | 1 |

Bactéricidie sur *St.aureus* ATCC 9144 selon le text décrit à la Pharmacopée Française.

Moyennes géométriques de 2 essais indépendants

| Temps de contact | Dilutions | 90% | 50% | 23% |
|---|---|---|---|---|
| 2' | n = 2 | 3,05 | 1,63 | 0,45 négligeable |
| 5' | n = 2 | 5,58 | 2,55 | 1,43 |

L'intérêt de cette formulation a été vérifié par un test clinique avec étude de la rémanence cutanée. En effet, sur une surface de 10 cm$^2$ chez cinq sujets n'ayant pas reçu de traitement antibiotique 15 jours au préalable, la flore cutanée a été étudiée à $T_0$, $T_{3j}$ et $T_{7j}$, puis une application du produit dilué à 50% dans l'eau a été réalisée pendant une minute.

Un rinçage à l'eau distillée stérile suivi d'un séchage avec une gaze stérile, a précédé un prélèvement de la surface lavée.

Ces lavages ont été réalisés aux temps $T_0$, $T_3$ jours et $T_7$ jours.

Le nombre d'unités formant colonies (UFC) par $cm_2$ a été calculé par sujet et par temps de prélèvement.

Le pourcentage de réduction se calcule selon la formule suivante par jour et par sujet :

$$\% = \frac{\text{Témoin - Essai}}{\text{Témoin}} \times 100$$

Témoin : nombre d'UFC / $cm^2$ le jour du test avant lavage

Essai : nombre d'UFC / $cm^2$ le jour du test après lavage.

Les résultats sont :

| Sujets | Temps | $T_0$ | $T_3$ | $T_7$ |
|--------|-------|-------|-------|-------|
| 1 | | 78 | 90 | 96 |
| 2 | | 60 | 90 | 77 |
| 3 | | 0 | 57 | 18 |
| 4 | | 88 | 89 | 91 |
| 5 | | 58,5 | 81,8 | 80 |
| m | | 56,9 | 81,8 | 72,4 |
| $\pm x \sigma n$ | | $\pm 30,5$ | $\pm 12,6$ | $\pm 28,0$ |

Moyennes et écarts-types des pourcentages de réduction du nombre d'UFC par $cm^2$ sur 5 sujets et sur 3 jours.

Nous voyons que le produit conserve une certaine rémanence après 3 jours et 7 jours d'application.

Il est évident que si le produit était utilisé quotidiennement (à $T_0$, T 1 jour, T 2 jours... jusqu'à T 7 jours), l'efficacité serait encore bien supérieure.

Lorsque les compositions selon l'invention sont présentées sous la forme de compositions cosmétiques, elles peuvent en particulier être utilisées comme :

- déodorant axilliaire, plantaire, ou buccal ;
- nettoyant et assainissant corporel pour peaux sensibles et peaux de bébé, ou
- produit d'hygiène intime externe.

Lorsque les compositions selon l'invention sont présentées sous la forme de médicaments dermatologiques, elles peuvent en particulier être utilisées pour le traitement des affections suivantes :

- acné, dont l'acné juvénile ;
- dermatite atopique ;
- toute pathologie cutanée nécessitant. un lavage (escarres, petites plaies...) ;
- assainissement des mains et pourtour des ongles, notamment dans les cas de périonyxis.

A l'occasion de la mise en oeuvre de différentes formulations des composiions selon l'invention, il est apparu que ces dernières étaient en quelque sorte autoprotégées, c'est-à-dire qu'il était inutile d'ajouter un quelconque conservateur traditionnel.

Il a également été constaté que l'efficacité antimicrobienne sur *St.aureus* variait en fonction de la concentration en :

- bétaïne : en effet celle-ci joue un rôle inhibiteur de l'efficacité (les cinétiques augmentent dès que les concentrations augmentent) ;
- épaississant : augmenter sa teneur contribue à l'augmentation de l'efficacité antimicrobienne.

Ainsi, pour une teneur minimale en bétaïne et maximale en épaississant, une excellente potentialisation a été obtenue en supprimant tout autre conservateur.

On indiquera ci-après à titre d'illustration quelques exemples de compositions selon l'invention. Ces diverses compositions illustrent notamment le fait que l'association synergique selon l'invention peut être combinée à différents autres principes actifs et excipients, et en particulier à une faible quantité de sulfate de cuivre.

EXEMPLE I : GEL MOUSSANT pour PEAU ATOPIOUE

| 1 | LIPOAMINOACIDE | 0,1 à 10,0g |
|---|---|---|
| 2 | Zn SO 4 | 0,1 à 5,0g |
| 3 | CU SO 4 | 0,1 à 0,5g |
| 4 | EXTRAIT d'AVOINE G1 | 0,1 à 3,0g |
| 5 | GLYCEROL | 0,1 à 5,0g |
| 6 | CROVOL PK 70 | 0,1 à 5,0g |
| 7 | CETIOL HE | 0,1 à 5,0g |
| 8 | DIMETHYLPOLYSILOXANE COPOLYOL | 0,1 à 5,0g |
| 9 | POLYQUART H 81 | 0,1 à 5,0g |
| 10 | GLUCAMATE DOE 120 | 0,1 à 5,0g |
| 11 | ELFACOS GT 282 S | 0,1 à 5,0g |
| 12 | ORAMK NS 10 | 0,1 à 2,0g |
| 13 | COCAMIDOPROPYL BETAINE | 1,0 à 20,0g |
| 14 | MONTEINE LCQ | 1,0 à 10,0g |
| 15 | TRIETHANOLAMINE QSP pH 5 | |
| 16 | CHLOR. SODIUM SUPER EPURE QSP VISCO | |
| 17 | EAU PURIFIEE QSP | 100,0g |

EXEMPLE II : DEODORANT BILLE

| 1 | ARLATONE 2121 | 2,0 à 10,0 g |
|---|---|---|
| 2 | ADIPATE d'ISOPROPYLE | 1,0 à 10,0 g |
| 3 | DECAMETHYL CYCLOPENTASILOXANE | 1,0 à 15,0 g |
| 4 | MONOSTEARATE de GLYCEROL | 1,0 g |
| 5 | PARFUM | 0,4 g |
| 6 | LIPOAMINOACIDE | 0,1 à 10,0 g |
| 7 | Zn SO 4 | 0,1 à 5,0 g |
| 9 | Cu SO 4 | 0,01 à 1,0 g |
| 10 | EAU PURIFIEE QSP | 100,0 g |

EXEMPLE III : STICK DEODORANT à pH NEUTRE

| 1 | DISORBENE LC | 1,0 à 2,0 g |
|---|---|---|
| 2 | HYDROXYPROPYLCELLULOSE MF | 0,1 à 2,0 g |
| 3 | DIPROPYLENE GLYCOL | 1,0 à 50,0 g |
| 4 | MACROGOL 300 | 1,0 à 20,0 g |
| 5 | PROPYLENE GLYCOL | 1,0 à 20,0 g |
| 6 | CARBONATE de PROPYLENE | 1,0 à 15,0 g |
| 7 | LIPOAMINOACIDE | 0,1 à 10,0 g |
| 8 | Zn SO 4 | 0,1 à 5,0g |
| 9 | Cu SO 4 | 0,01 à 1,0g |
| 10 | EAU PURIFIEE QSP | 100,0 g |

EXEMPLE IV : SPRAY DEODORANT

| 1 | FLUIDE DC 344 | 6,0 à 30,0 g |
|---|---|---|
| 2 | SMECTITE | 0,1 à 2,0 g |
| 3 | ACIDECITRIQUEMONOHYDR. QSP pH 7,8 | |
| 4 | PARFUM | 0,30 g |
| 5 | DMDM HYDANTOIN | 0,10 g |
| 6 | LIPOAMINOACIDE | 0,1 à 0,10 g |
| 7 | Zn SO 4 | 0,1 à 5,0 g |
| 8 | Cu SO 4 | 0,01 à 1,0 g |
| 9 | EAU PURIFIEE QSP | 100,0 g |

EXEMPLE V : LINGETTES BEBE - NETTOYANTES et ASSAINISSANTES

| 1 | SEPIGEL 501 | 1,0 à 2,0 g |
|---|---|---|
| 2 | PARFUM | 0,3 g |
| 3 | LIPOAMINOACIDE | 0,1 à 5,0 g |
| 4 | Zn SO 4 | 0,1 à 5,0 g |
| 5 | CuS0 4 | 0,01 à 1,0 g |
| 6 | EAU PURIFIEE QSP | 97,49 g |

EXEMPLE VI : DEODORANT STICK FRAICHEUR

| 1 | EXTRAIT de FRAISIER | 0,50 g |
|---|---|---|
| 2 | SENECIOATE de CITRONELLYLE | 0,1 à 0,30g |
| 3 | ACETATE d'ALPHA TOCOPHEROL | 0,1 à 2,0 g |
| 4 | PROPYLENE GLYCOL | 20,0 à 50,0 g |
| 5 | GLYCERINE CODEX | 10,0 à 20,0 g |
| 6 | ALCOOL CETEARYLIQUE 50 OE | 3,0 à 6,0 g |
| 7 | STEARATE de SODIUM | 2,0 à 7,0 g |
| 8 | PARFUM | 0,7 g |
| 9 | LIPOAMINOACIDE | 0,1 à 10,0 g |
| 10 | Zn SO 4 | 0,1 à 5,0 g |
| 11 | Cu SO 4 | 0,01 à 1,0 g |
| 12 | EAU PURIFIEE QSP | 100,0 g |

EXEMPLE VII : GEL de TOILETTE sans RINCAGE, ANTIBACTERIEN

| 1 | TRIGLYCERIDE CAPRIQUE/CAPRYLIQUE | 0,1 à 6,0 g |
|---|---|---|
| 2 | FINSOLV TN | 0,1 à 6,0 g |
| 3 | ACIDE SORBIQUE | 0,10 g |
| 4 | PENIULEN | 0,1 à 0,5 g |
| 5 | CARBOPOL | 0,1 à 0,5 g |
| 6 | ORAMIXNS 10 | 0,1 à 5,0 g |
| 7 | EDETATE DISODIQUE Ph. Eur. | 0,2 g |

(suite)

| 8 | TRIETHANOLAMINE QSP pH | |
|---|---|---|
| 9 | PARFUM | 0,06 g |
| 10 | LIPOAMINOACIDE | 0,1 à 10,0 g |
| 11 | Zn SO 4 | 0,1 à 5,0 g |
| 12 | Cu SO 4 | 0,01 à 1,0 g |
| 13 | EAU PURIFIEE QSP | 100,0 g |

EXEMPLE VIII : CREME ANTIBACTERIENNE pour DARTRES et PEAUX ATOPIOUES

| 1 | LIPOAMINOACIDE | 1 à 10,0 g |
|---|---|---|
| 2 | Zn SO 4 | 0,1 à 5,0 g |
| 3 | Cu SO 4 | 0,1 à 5,0g |
| 4 | HOSTACERIN WO | 10,0 g |
| 5 | PARAFFINE LIQUIDE | 5,0 à 15,0 g |
| 6 | CIRE d'ABEILLE EXTRA BLANCHE | 2,0 g |
| 7 | VASEUNE BLANCHE | 5,0 à 15,0 g |
| 8 | PALMITATE d'ISOPROPYLE | 5,0 à 15,0 g |
| 9 | PCL HUILEUX | 1,0 à 5,0 g |
| 10 | PHENONIP | 0,5 g |
| 11 | PARAHYDROXYBENZOATE de PROPYLE | 0,15 g |
| 12 | GLYCEROL | 4,0 g |
| 13 | EAU PURIFIEE QSP | 100,0 g |

EXEMPLE IX : GEL MOUSSANT

| 1 | POLYQUART H 81 | 1,0 g |
|---|---|---|
| 2 | MIRANOL C2M | 10,0 à 35,0 g |
| 3 | POLYSORBATE 20 | 1,0 à 10,0 g |
| 4 | COCAMIDOPROPYL BETAINE | 1,0 à 20,0 g |
| 5 | ORAMIXNS 10 | 1,0 à 20,0 g |
| 6 | ELEACOS GT 282 S | 1,0 à 3,0 g |
| 7 | GLYCEROL | 1,0 g |
| 8 | ACIDE CITRIQUE MONOHYDRATE Ph. Eur. | 0,8 g |
| 9 | LIPOAMINOACIDE | 0,1 à 10,0 g |
| 10 | Zn SO 4 | 0,1 à 5,0 g |
| 11 | Cu SO 4 | 0,1 à 5,0 g |
| 12 | EAU PURIFIEE QSP | 100,0 g |

EXEMPLE X : EMULSION MOUSSANTE 2 en 1- CORPOREL ANTIBACTERIEN

| 1 | LAURETH SULFATE | 10 à 20,0 g |
|---|---|---|
| 2 | CRODASINIC LS 30 | 1 à 25,0 g |
| 3 | COCAMIDOPROPYL BETAINE | 1 à 15,0 g |
| 4 | COMPERLAN LMD | 1 à 5,0 g |
| 5 | ACIDE MYRISTIQUE | 0,5 à 8,0 g |

(suite)

| 6 | TRIETHANOLAMINE | 0,1 à 0,5 g |
|---|---|---|
| 7 | CETIOLHE | 1 à 15,0 g |
| 8 | ARLAMOL HD | 1 à 10,0 g |
| 9 | 2-OCTYIDODECANOL | 1 à 10,0 g |
| 10 | PARFUM | 0,4 g |
| 11 | UPOAMINOACIDE | 0,1 à 10,0 g |
| 12 | Zn SO 4 | 0,1 à 5,0 g |
| 13 | Cu SO 4 | 0,01 à 1,0 g |
| 14 | EAU PURIFIEE QSP | 100,0 g |

EXEMPLE XI : EMULSION MOUSSANTE 2 en 1- CORPOREL ANTIBACTERIEN

| 1 | LAURETH SULFATE | 10 à 20,0 g |
|---|---|---|
| 2 | CRODASINIC LS 30 | 1 à 25,0 g |
| 3 | COCAMIDOPROPYL BETAINE | 1 à 15,0 g |
| 4 | COMPERLAN LMD | 1 à 5,0 g |
| 5 | ACIDE MYRISTIQUE | 0,5 à 8,0 g |
| 6 | TRIETHANOLAMINE | 0,1 à 0,5 g |
| 7 | CETIOL HE | 1 à 15,0 g |
| 8 | ARLAMOL HD | 1 à 10,0 g |
| 9 | 2-OCIYLDODECANOL | 1 à 10,0 g |
| 10 | PARFUM | 0,4 g |
| 11 | LIPOAMINOACIDE | 0,1 à 10,0 g |
| 12 | Zn SO 4 0,1 à 5,0 g | |
| 13 | Cu SO 4 0,01 à 1,0 g | |
| 14 | EAU PURIFIEE QSP | 100,0 g |

EXEMPLE XI : GEL MOUSSANT ANTIBACTERIEN, KERATOLYTIOUE et ANTIACNEIQUE

| 1 | GLYCEROL | 1 à 3,0 g |
|---|---|---|
| 2 | CETIOL HE | 1 à 4,0 g |
| 3 | ACIDE SALICYLIQUE PULVERISE | 1 à 4,0 g |
| 4 | MIRANOL C2M | 5 à 25,0 g |
| 5 | ORAMIX NS 10 | 5 à 15,0 g |
| 6 | MONTEINE LCQ | 1 à 15,0 g |
| 7 | ELFACOS GT282 S | 1 à 3,0 g |
| 8 | LIPOAMINOACIDE | 0,1 à 10,0 g |
| 9 | Zn SO 4 0,1 à 5,0 g | |
| 10 | Cu SO 4 0,01 à 1,0 g | |
| 11 | EAU PURIFIEE QSP | 100,0 g |

EXEMPLE XII : PAIN de TOILETTE sans SAVON ANTIBACTERIEN

| 1 | HUILE d'AMANDE DOUCE RAFFINEE | 0,50 g |
|---|---|---|
| 2 | VITAMINE F ESTER GLYCERIQUE | 0,10 g |

(suite)

| 3 | PARFUM | 0,30 g |
|---|---|---|
| 4 | LIPOAMINOACIDE | 0,1 g à 5,0 g |
| 5 | Zn SO 4 | 0,1g à 5,0 g |
| 6 | Cu SO 4 | 0,01 g à 1,0 g |
| 7 | BASE SYNDET COCOYL ISETHIONATE HEMISULFOSUCCINATE QSP | 100,0 g |

EXEMPLE XIII : <u>BAIN de BOUCHE ASSAINISSANT et DEODORANT</u>

| 1 | ESSENCE de MENTHE | 0,60 g |
|---|---|---|
| 2 | ESSENCE de BADIANE | 0,20 g |
| 3 | ALCOOL à 95 ° | 57,310 g |
| 4 | LIPOAMINOACIDE | 0,1 g à 10,0 g |
| 5 | Zn SO 4 0,1 g à 5,0 g | |
| 6 | Cu SO 4 0,01 g à 1,0 g | |
| 7 | EAU DISTILLEE QSP | 100,0 g |

EXEMPLE XIV : <u>DERMO PAIN PEAUX DELICATES</u>

| 1 | PARFUM 0,30 g | |
|---|---|---|
| 2 | LIPOAMINOACIDE | 0,1 g à 3,0 g |
| 3 | Zn SO 4 0,1 g à 5,0 g | |
| 4 | CU ZO 4 0,01 g à 1,0 g | |
| 5 | BASE SYNDET QSP | 100,0 g |

EXEMPLE XV : <u>SOLUTION ANTI TRANSPIRANTE et DEODORANTE</u>

| 1 | EUMULGIN B1 | 0,5g à 2,0 g |
|---|---|---|
| 2 | CUTINA MD | 0,5 g à 2,0 g |
| 3 | DICAPRYL ETHER | 5 g à 15,0 g |
| 4 | LIPOAMINOACIDE | 0,1 g à 10,0 g |
| 5 | Zn SO 4 | 0,1 g à 5,0 g |
| 6 | Cu SO 4 | 0,01g à 1,0 g |
| 7 | SELS d'ALUMINIUM | 1,0 g à 25,0 g |
| 8 | EAU PURIFIEE QSP 100,0 g | 83,99 g |

EXEMPLE XVI : <u>SOLUTION ANTI TRANSPIRANTE et DEODORANTE</u>

| 1 | EUMULGIN B1 | 0,5g à 2,0 g |
|---|---|---|
| 2 | CUTINA MD | 0,5 g à 2,0 g |
| 3 | DICAPRYL ETHER | 5 g à 15,0 g |
| 4 | LIPOAMINOACIDE | 0,1 g à 10,0 g |
| 5 | Zn SO 4 | 0,1 g à 5,0 g |
| 6 | Cu SO 4 | 0,01 g à 1,0 g |

(suite)

| | | |
|---|---|---|
| 7 | UROFORMINE | 0,1 g à 5,0 g |
| 8 | EAU PURIFIEE QSP 100,0 g | 83,99 g |

**Revendications**

1. Composition antibactérienne, **caractérisée en ce qu'**elle contient une association synergique d'une acylglycine en $C_6$ à $C_{10}$ avec un sel de zinc.

2. Composition antibactérienne selon la revendication 1, **caractérisée en ce que** l'acylglycine est l'octanoylglycine.

3. Composition antibactérienne selon l'une des revendications 1 et 2, **caractérisée en ce que** le sel de zinc est le sulfate de zinc.

4. Composition antibactérienne selon l'une des revendications 1 à 3, **caractérisée en ce que** l'acylglycine est présente à raison de 0,1 à 10% en poids par rapport au poids total de la composition.

5. Composition antibactérienne selon la revendication 4, **caractérisée en ce que** l'acylglycine est présente à raison de 2 à 4% en poids par rapport au poids total de la composition.

6. Composition antibactérienne selon l'une des revendications 1 à 5, **caractérisée en ce que** le sel de zinc est présent à raison de 0,05 à 5% en poids par rapport au poids total de la composition.

7. Composition selon la revendication 6, **caractérisée en ce que** le sel de zinc est présent à raison de 0,5 à 1%.

8. Composition selon l'une des revendications 1 à 7, **caractérisée en ce qu'**elle contient un support pharmaceutiquement acceptable en vue de son application sur la peau en tant que médicament topique.

9. Composition selon l'une des revendications 1 à 7, **caractérisée en ce qu'**elle contient un support cosmétiquement acceptable en vue de son application sur la peau en tant que produit cosmétique.

10. Utilisation d'une association synergique telle que définie dans l'une des revendications 1 à 7 pour la fabrication d'un médicament destiné à traiter des affections cutanées d'origine bactérienne.

11. Utilisation d'une association synergique telle que définie dans l'une des revendications 1 à 7 pour la fabrication d'un produit cosmétique, en particulier un produit d'hygiène corporelle.

12. Composition selon la revendication 9, **caractérisée en ce qu'**elle se présente sous la forme d'un gel moussant répondant à la formulation suivante :

| | | |
|---|---|---|
| 1 | LIPOAMINOACIDE | 0,1 à 10,0 g |
| 2 | Zn SO 4 | 0,1 à 5,0 g |
| 3 | Cu SO 4 | 0,1 à 0,5 g |
| 4 | EXTRAIT d'AVOINE G1 | 0,1 à 3,0 g |
| 5 | GLYCEROL | 0,1 à 5,0 g |
| 6 | CROVOL PK 70 | 0,1 à 5,0 g |
| 7 | CETIOL HE | 0,1 à 5,0 g |
| 8 | DIMETHYLPOLYSILOXANE COPOLYOL | 0,1 à 5,0 g |
| 9 | POLYQUART H 81 | 0,1 à 5,0 g |
| 10 | GLUCAIvIATE DOE 120 | 0,1 à 5,0 g |
| 11 | ELFACOS GT 282 S | 0,1 à 5,0 g |
| 12 | ORAMIX NS 10 | 0,1 à 2,0 g |
| 13 | COCAMIDOPROPYL BETAINE | 1,0 à 20,0 g |
| 14 | MONTEINE LCQ | 1,0 à 10,0 g |

(suite)

| 15 | TRIETHANOLAMINE QSP pH 5 | |
|----|--------------------------|--------|
| 16 | CHLOR. SODIUM SUPER EPURE QSP VISCO | |
| 17 | EAU PURIFIEE QSP | 100,0 g |

**Patentansprüche**

1.  Antibakterielle Zusammensetzung, **dadurch gekennzeichnet**, dass sie eine synergistische Vereinigung eines $C_6$- bis $C_{10}$-Acylglycins mit einem Zinksalz enthält.

2.  Antibakterielle Zusammensetzung nach Anspruch 1, **dadurch gekennzeichnet**, daß das Acylglycin Octanoylglycin ist.

3.  Antibakterielle Zusammensetzung nach einem der Ansprüche 1 und 2, **dadurch gekennzeichnet**, daß das Zinksalz Zinksulfat ist.

4.  Antibakterielle Zusammensetzung nach einem der Ansprüche 1 bis 3, **dadurch gekennzeichnet**, daß das Acylglycin in einem Verhältnis von 0,1 bis 10 Gew.-%, bezogen auf das Gesamtgewicht der Zusammensetzung, vorliegt.

5.  Antibakterielle Zusammensetzung nach Anspruch 4, **dadurch gekennzeichnet**, daß das Acylglycin in einem Verhältnis von 2 bis 4 Gew.- %, bezogen auf das Gesamtgewicht der Zusammensetzung, vorliegt.

6.  Antibakterielle Zusammensetzung nach einem der Ansprüche 1 bis 5, **dadurch gekennzeichnet**, daß das Zinksalz in einem Verhältnis von 0,05 bis 5 Gew.-%, bezogen auf das Gesamtgewicht der Zusammensetzung, vorliegt.

7.  Zusammensetzung nach Anspruch 6, **dadurch gekennzeichnet**, daß das Zinksalz in einem Verhältnis von 0,5 bis 1 % vorliegt.

8.  Zusammensetzung nach einem der Ansprüche 1 bis 7, **dadurch gekennzeichnet,** daß sie einen Träger enthält, der im Hinblick auf ihre Anwendung auf der Haut als topisches Medikament pharmazeutisch annehmbar ist.

9.  Zusammensetzung nach einem der Ansprüche 1 bis 7, **dadurch gekennzeichnet,** daß sie einen Träger enthält, der im Hinblick auf ihre Anwendung auf der Haut als kosmetisches Produkt kosmetisch annehmbar ist.

10. Verwendung einer synergistischen Vereinigung, wie in einem der Ansprüche 1 bis 7 definiert, für die Herstellung eines Medikaments, welches dazu bestimmt ist, Hautleiden von bakteriellem Ursprung zu behandeln.

11. Verwendung einer synergistischen Vereinigung, wie in einem der Ansprüche 1 bis 7 definiert, für die Herstellung eines kosmetischen Produkts, insbesondere eines Produkts der Körperhygiene.

12. Zusammensetzung nach Anspruch 9, **dadurch gekennzeichnet**, daß sie in Form eines schäumenden Gels vorliegt, das der folgenden Formulierung entspricht:

| 1 | Lipoaminosäure | 0,1 bis 10,0 g |
|----|------------------------------|----------------|
| 2 | $ZnSO_4$ | 0,1 bis 5,0 g |
| 3 | $CuSO_4$ | 0,1 bis 0,5 g |
| 4 | Haferextrakt G1 | 0,1 bis 3,0 g |
| 5 | Glycerin | 0,1 bis 5,0 g |
| 6 | Crovol PK 70 | 0,1 bis 5,0 g |
| 7 | Cetiol HE | 0,1 bis 5,0 g |
| 8 | Dimethylpolysiloxan-co-Polyol | 0,1 bis 5,0 g |
| 9 | Polyquart H 81 | 0,1 bis 5,0 g |
| 10 | Glucamat DOE 120 | 0,1 bis 5,0 g |

**EP 0 869 800 B1**

(fortgesetzt)

| 11 | Elfacos GT 282 S | 0,1 bis 5,0 g |
|---|---|---|
| 12 | Oramix NS 10 | 0,1 bis 2,0 g |
| 13 | Cocamidopropylbetain | 1,0 bis 20,0 g |
| 14 | Monteine LCQ | 1,0 bis 10,0 g |
| 15 | Triethanolamin q.s.p. pH 5 | |
| 16 | Natriumchlorid, supergereinigt q.s.p. Viskosität | |
| 17 | gereinigtes Wasser q.s.p. | 100,0 g |

**Claims**

1. Antibacterial composition, **characterized in that** it contains a synergetic association of a $C_6$ to $C_{10}$ acylglycine with a zinc salt.

2. Antibacterial composition according to Claim 1, **characterized in that** the acylglycine is octanoylglycine.

3. Antibacterial composition according to either of Claims 1 and 2, **characterized in that** the zinc salt is zinc sulphate.

4. Antibacterial composition according to one of Claims 1 to 3, **characterized in that** the acylglycine is present in an amount of 0.1 to 10% by weight relative to the total weight of the composition.

5. Antibacterial composition according to Claim 4, **characterized in that** the acylglycine is present in an amount of 2 to 4% by weight relative to the total weight of the composition.

6. Antibacterial composition according to one of Claims 1 to 5, **characterized in that** the zinc salt is present in an amount of 0.05 to 5% by weight relative to the total weight of the composition.

7. Composition according to Claim 6, **characterized in that** the zinc salt is present in an amount of 0.5 to 1%.

8. Composition according to one of Claims 1 to 7, **characterized in that** it contains a carrier which is pharmaceutically acceptable for its application to the skin as a topical medicament.

9. Composition according to one of Claims 1 to 7, **characterized in that** it contains a carrier which is cosmetically acceptable for its application to the skin as a cosmetic product.

10. Use of a synergetic association as defined in one of Claims 1 to 7 for the manufacture of a medicament intended for treating skin conditions of bacterial origin.

11. Use of a synergetic association as defined in one of Claims 1 to 7 for the manufacture of a cosmetic product, in particular a product for body hygiene.

12. Composition according to Claim 9, **characterized in that** it is provided in the form of a foam gel corresponding to the following formula:

| 1 | LIPOAMINO ACID | 0.1 to 10.0 g |
|---|---|---|
| 2 | ZnSO4 | 0.1 to 5.0 g |
| 3 | CuSO4 | 0.1 to 0.5 g |
| 4 | OAT EXTRACT G1 | 0.1 to 3.0 g |
| 5 | GLYCEROL | 0.1 to 5.0 g |
| 6 | CROVOL PK 70 | 0.1 to 5.0 g |
| 7 | CETIOL HE | 0.1 to 5.0 g |
| 8 | DIMETHYLPOLYSILOXANE COPOLYOL | 0.1 to 5.0 g |
| 9 | POLYQUART H 81 | 0.1 to 5.0 g |

13

(continued)

| 10 | GLUCAMATE DOE 120 | 0.1 to 5.0 g |
| 11 | ELFACOS GT 282 S | 0.1 to 5.0 g |
| 12 | ORAMIX NS 10 | 0.1 to 2.0 g |
| 13 | COCAMIDOPROPYL BETAINE | 1.0 to 20.0 g |
| 14 | MONTEINE LCQ | 1.0 to 10.0 g |
| 15 | TRIETHANOLAMINE QS pH 5 | |
| 16 | SUPER PURE SODIUM CHLORIDE QS VISCO | |
| 17 | PURIFIED WATER QS | 100.0 g |